# EUROPÄISCHE PATENTSCHRIFT

(11) **EP 1 056 401 B1**
(45) Veröffentlichungstag und Bekanntmachung des Hinweises auf die Patenterteilung: **03.11.2004**
(21) Anmeldenummer: 99907538.5
(22) Anmeldetag: 11.02.1999
(51) Int. Cl.: A61B 17/32

(54) **VORRICHTUNG ZUR BEHANDLUNG VON KÖRPERGEWEBE MITTELS ULTRASCHALL**
DEVICE FOR ULTRASONIC TREATMENT OF BODY TISSUES
DISPOSITIF POUR TRAITER DES TISSUS CORPORELS AU MOYEN D'ULTRASONS

(30) Priorität: 18.02.1998 DE 19806718
(43) Veröffentlichungstag der Anmeldung: 06.12.2000
(73) Patentinhaber: Storz-Endoskop GmbH, 8200 Schaffhausen (CH)
(72) Erfinder: NOVAK, Pavel, CH-8207 Schaffhausen (CH); HENES, Rudolf, CH-8200 Schaffhausen (CH); Krattiger, Beat, 8222 Beringen (CH)
(74) Vertreter: Weller, Wolfgang, Dr.
(86) Internationale Anmeldenummer: PCT/EP1999/000892
(87) Internationale Veröffentlichungsnummer: WO 1999/042040

(56) Entgegenhaltungen:
- US-A- 4 838 853
- US-A- 5 167 619
- US-A- 5 176 677
- US-A- 5 403 307
- US-A- 5 674 235

## Beschreibung

Die Erfindung betrifft eine Vorrichtung zum Behandeln von Körpergewebe mittels Ultraschall, mit einem Generator und mit einer Einrichtung zum Übertragen des Ultraschalls auf das Gewebe, die eine Hohlsonde zum Absaugen des behandelten Gewebes aufweist, wobei im Bereich des distalen Endes der Hohlsonde eine Schneideklinge vorgesehen ist.

Eine derartige Vorrichtung ist aus der US-A-4 838 853 bekannt.

Eine vergleichbare Vorrichtung ist auch aus der US-A-5 674 235 bekannt.

Ultraschall wird nicht nur im diagnostischen Bereich zur Sichtbarmachung von Körperstrukturen mit unterschiedlicher Dichte sondern auch zu therapeutischen Zwecken eingesetzt. Dazu wird der Ultraschall entweder von außen durch die Haut appliziert oder mit entsprechenden laparoskopischen Instrumenten, ggf. unter endoskopischer Kontrolle, im Körperinneren direkt an der Stelle des zu behandelnden Gewebes appliziert.

Durch die Ultraschall-Behandlung kommt es zum einen zu einer Erwärmung des Gewebes und damit zu dessen Denaturierung, der sogenannten (Thermo-) Koagulation.

Neben der Wärmeentwicklung hat der Ultraschall eine mechanische Wirkung in Form von sog. Kavitation. Schallwellen breiten sich nämlich in einem Medium als sich periodisch ändernde Dichteschwankungen des Mediums aus. Ein Volumenelement des Mediums wird abwechselnd verdichtet (Überdruck) und expandiert (Unterdruck). Der Unterdruck kann in einer Flüssigkeit, beispielsweise im Zytoplasma der Zellen oder in der Gewebsflüssigkeit, zur Dampfblasenbildung führen, wodurch Zellen und Gewebe zerstört werden.

Eine derartige therapeutische Ultraschallbehandlung ist dazu geeignet, weiche Gewebe wie beispielsweise Fettgewebe zu desintegrieren, d.h. aufzulösen. Dabei werden im Allgemeinen Ultraschallwellen im Bereich von 26 kHz eingesetzt.

Das koagulierte und/oder desintegrierte Gewebe wird durch eine rohrförmige Hohlsonde abgesaugt, wobei meist außerdem eine Spüleinrichtung zum Durchspülen vorgesehen ist. Die Spülflüssigkeit wird durch einen Hohlraum zwischen der Hohlsonde und einem Außenschaft geleitet. Die mit der Spülflüssigkeit vermischten Zell- bzw. Gewebereste werden durch die Hohlsonde abgesaugt.

Derartige Ultraschallbehandlungen werden vor allem bei der Zerstörung von tumorösem Gewebe eingesetzt. Insbesondere in der Leberchirurgie werden Ultraschallbehandlungen angewandt, da mit dieser Technik skeletosiniert werden kann, d.h. von einen Organ wie die Leber können lediglich Gewebeteile abgelöst werden ohne dabei Blutgefäße des Organs zu zertrennen, bzw. zu zerstören.

Da mittels Ultraschall jedoch nur extrem weiches Gewebe in lokkeren Zellverbänden zerstört werden kann, reicht eine Ultraschallbehandlung von festeren Tumorgeweben oder anderen zu entfernenden festen Geweben nicht aus. Dazu müssen dann weitere scharfe Schneidegeräte, Skalpelle o.ä. eingesetzt werden, was die Operation zusätzlich erschwert.

Weitere medizinische Instrumente, bei denen Ultraschall zur Anwendung kommt, sind chirurgische Messer oder Skalpelle, die mittels Ultraschall in Schwingungen versetzt werden.

Ein derartiges "ultraschallgetriebenes" chirurgisches Messer für laparoskopische Anwendungen ist beispielsweise in der WO 93/14708 beschrieben. Bei diesem Instrument ist ein Messer verschieblich im Inneren eines Schaftes angeordnet, aus dem es während der Operation herausgeschoben werden kann. Ultraschallenergie wird direkt und ausschließlich auf das Messer übertragen, das dadurch zum Schwingen angeregt wird.

Eine therapeutische Applikation von Ultraschall zur Desintegration von Gewebe ist mit diesem chirurgischen Schneidegerät nicht möglich. Außerdem besteht keine Möglichkeit, die abgeschnittenen Gewebeteile aus dem Operationsfeld zu entfernen. Darüber hinaus ist diese Vorrichtung auf Durchmesser von größer als einem Zentimeter beschränkt.

In einer Weiterentwicklung dieses chirurgischen Messers, die in der US 5,346,502 beschrieben ist, wird deshalb ein ähnliches, durch Ultraschall zum Vibrieren bringbares chirurgisches Messer beschrieben, bei dem ein Außendurchmesser des Schafts von 5 mm erreicht wird. Dieses Instrument ist jedoch in seiner Herstellung extrem kompliziert, da der Schaft aus PTFE hergestellt und mehreren Ausdehnungs- und Schrumpfprozessen unterworfen werden muß. Außerdem ist auch bei diesem Messer keine Ultraschallapplikation zur Zerstörung von weichem Gewebe möglich.

Aus der DE 40 42 435 C2 ist eine Ultraschallbehandlungsvorrichtung mit einem Handstück, welches einen Ultraschalloszillator aufweist, und eine Mehrzahl von Sonden, welche mit dem Handstück verbindbar sind, bekannt. Dadurch ist es möglich, eine Ultraschallvorrichtung bereitzustellen, welche bei geringen Anschaffungs- und Unterhaltskosten gleichzeitig für unterschiedlichste Einsatzzwecke geeignet ist.

Aus der EP 0 384 672 A2 ist eine Ultraschallhohlsonde bekannt, bei der die distale Saugöffnung in ihrer Form und Größe verändert werden kann, um eine jeweils optimale Saugkraft zum Absaugen des behandelten Gewebes zu erhalten.

Aus der EP 0 238 667 A1 ist ein Ultraschallinstrument bekannt, dessen distales Ende löffelartig ausgebildet ist. Ein mittiger Spülkanal dient dazu, eine Flüssigkeit zum löffelartig ausgebildeten distalen Ende zuzuführen.

Aus der US 5 188 102 A1 ist ein durch Ultraschall zum Vibrieren bringbares chirurgisches Messer beschrieben, das mittig mit einem Spülkanal versehen ist, über den eine Spülflüssigkeit, insbesondere unter einem Winkel von 5 bis 90° bezüglich der Schneideebene, zum distalen Ende zugeführt werden kann.

Aus der US 5 047 043 A1 ist ein mit Ultraschall betriebenes chirurgisches Messer beschrieben, in dem ein Endoskop eingeschoben ist, um das distale Ende visuell beobachten zu können.

Aus der EP 0 695 535 A1 ist ein mit Ultraschall betriebenes Greifinstrument beschrieben, das das Gewebe, das durch den Ultraschall abgelöst wurde, ergreifen kann.

Aus der US 5 322 055 A1 ist eine mit Ultraschall betriebene Vorrichtung bekannt, die zum Schneiden und zum Koagulieren geeignet ist. Zum Koagulieren ist am distalen Ende eine Klemmvorrichtung vorgesehen, über die zwischen ein bewegliches Maulteil und ein ortsfestes Maulteil das zu koagulierende Gewebe gebracht werden kann.

Aufgabe der vorliegenden Erfindung ist es, eine Vorrichtung zu schaffen, mit der neben der Applikation von Ultraschall eine weitere Möglichkeit der Gewebemanipulation zur Verfügung gestellt wird.

Erfindungsgemäß wird die Aufgabe dadurch gelöst, daß das distale Ende der Hohlsonde eine Einrichtung zum Greifen und/oder Durchtrennen mit einer ersten und einer zweiten Backe aufweist.

Diese Maßnahme hat den Vorteil, daß die erfindungsgemäße Vorrichtung um zwei weitere Funktionen erweitert wird, die bei Operationen, bei denen Gewebe entfernt werden muß, oft benötigt werden: dem Ergreifen beispielsweise von Gefäßen, Nerven oder ähnlichem, und dem Durchtrennen derartiger Gewebestrukturen. Beim Durchtrennen von Adern ist vorteilhaft, daß die durch den Ultraschall erzeugte Wärmeentwicklung gleichzeitig auch zu einer Koagulation und damit zu einer Stillung der Blutung führt.

Die Greif- und Durchtrennungsfunktion ist besonders dann erforderlich, wenn besonders feste Gewebebestandteile entfernt oder durchschnitten bzw. abgetrennt werden müssen.

Wenn die Ultraschallquelle abgeschaltet ist, wird die erfindungsgemäße Vorrichtung durch die an dem distalen Ende der Hohlsonde angebrachten beiden Backen zu einer Klemme oder Klammer, mit der Blutgefäße auch ohne Durchtrennung abgeklemmt werden können.

Bei herkömmlichen Ultraschallgeräten zur therapeutischen Behandlung dient die Hohlsonde lediglich dazu, die koagulierten und verflüssigten Zell- und Gewebebestandteile aufzusaugen und abzuführen, gegebenenfalls ferner um Spülflüssigkeit in das Operationsfeld einzuspritzen und wieder aufzusaugen.

Indem am distalen Ende der Hohlsonde, also der zum Aufsaugen offenen Sondenspitze, eine Schneideklinge vorgesehen ist, wird auf einfache Art und Weise ein multifunktionelles medizinisches Instrument zur Verfügung gestellt, mit dem die weichen Gewebebestandteile durch Ultraschall desintegriert, abgesaugt und gleichzeitig festere, gegenüber Ultraschall unempfindliche Gewebeteile abgeschnitten und ebenfalls abgesaugt werden können.

Hierbei wird der Effekt ausgenutzt, daß auch bei einer therapeutischen Ultraschallanwendung das im Operationsfeld liegende distale Ende der Hohlsonde zum Vibrieren angeregt wird. Die vibrierende Schneideklinge hat hervorragende Schneideeigenschaften, und zwar auch dann, wenn die Klinge selbst vergleichsweise stumpf ist.

Die zur Zerstörung von Geweben geeigneten Funktionen der erfindungsgemäßen Vorrichtung werden ferner durch die Wärmeerzeugung der therapeutischen Ultraschallwellen unterstützt, so daß es mit einem einzigen Instrument möglich ist, gleichzeitig durch Ultraschallbehandlung des Gewebes zu desintegrieren, durch die Schneideklinge abzuschneiden und/oder abzuschaben und außerdem zu koagulieren, wobei ständig abgelöste Zell- bzw. Gewebebestandteile aus dem Operationsfeld entfernt werden.

Es versteht sich, daß die Schneideklinge auch zum Separieren von festen oder weichen Gewebebestandteilen eingesetzt werden kann, z.B. zum Separieren von Fettgewebe von subkutanem Gewebe. Unter diesen Umständen ist die erfindungsgemäße Vorrichtung als Schneide- und Absaugvorrichtung zu gebrauchen.

Die erfindungsgemäße Vorrichtung vereinigt in sich somit mehrere Funktionen, die bei den genannten Anwendungen direkt im Operationsfeld benötigt werden, und spart somit dem Operateur ein häufiges Wechseln der chirurgischen Geräte. Derartige Operationen sind somit wesentlich einfacher und schneller durchzuführen als es bisher möglich war.

Die Multifunktionalität der Vorrichtung wird dabei mit sehr einfachen Mitteln erreicht, nämlich durch die Ausstattung des proximalen Hohlsondenendes mit einer Schneideklinge.

Die der Erfindung zugrunde liegende Aufgabe wird somit vollkommen gelöst.

In einer vorteilhaften Ausgestaltung ist die Schneideklinge integraler Bestandteil der Hohlsondenwand.

Diese Maßnahme hat den Vorteil, daß die Schneideklinge besonders einfach hergestellt werden kann, nämlich gleichzeitig mit der Hohlsonde. Außerdem wird durch diese Maßnahme eine gute Beständigkeit erreicht, da Mittel zum Befestigen der Schneideklinge am distalen Ende der Hohlsonde wegfallen.

In einer besonders vorteilhaften Ausgestaltung der Erfindung ist die Schneideklinge als Fortsatz der Hohlsondenwand ausgebildet.

Die Schneideklinge kann dabei flächig oder gebogen sein, sie kann sich verjüngen oder verbreitern, je nach der gewünschten Anwendung.

Die Ausbildung der Schneideklinge als Fortsatz hat den wesentlichen Vorteil, daß die erfindungsgemäße Vorrichtung eine weitere Funktion erhält, nämlich auch als Spatel eingesetzt werden kann. Die Schneideklinge, die relativ stumpf ausgebildet sein kann, kann nämlich dann, wenn die Ultraschallquelle abgeschaltet ist, als Spatelfläche zum Abschaben von Gewebe eingesetzt werden. Das abgeschabte Gewebe befindet sich auf dem flächigen Fortsatz in einer Position direkt vor der Öffnung der Hohlsonde und kann damit unmittelbar abgesaugt werden.

Sobald der Ultraschallgenerator eingeschaltet wird, wird der Fortsatz in Schwingungen versetzt und hat dann die Wirkung einer scharfen Schneideklinge.

Dabei ist die Schneideklinge vorteilhafterweise exponiert und erreicht das Gewebe, das abgeschnitten werden soll, besonders gut.

In einer weiteren Ausgestaltung der Erfindung ist die erste Backe der Einrichtung zum Greifen und/oder Durchtrennen durch die Schneideklinge gebildet.

Diese Maßnahme hat den Vorteil, konstruktiv besonders einfach zu sein, da nur die zweite Backe zusätzlich am distalen Ende der Hohlsonde angebracht werden muß.

In einer weiteren Ausgestaltung der Erfindung ist die zweite Backe der Einrichtung zum Greifen und/oder Durchtrennen entlang der Längsachse der Hohlsonde verschiebar, besteht aus einem superelastischen Material und ist in einem aus der Hohlsonde herausgeschobenen Zustand von ersten Backe weggebogen und bewegt sich beim Hineinziehen in die Hohlsonde zu der ersten Backe hin.

Das Verschieben der Backe entlang der Längsachse kann dabei beispielsweise durch ein von der proximalen Seite der Vorrichtung bedienbares Betätigungselement durch Zug hervorgerufen werden.

Das superelastische Material kann beispielsweise Nitinol sein, eine Nickel-Titan-Legierung, die ein sogenanntes "Memorymaterial" ist. Dieses Material hat die Eigenschaft, auch nach Verformung immer wieder in seine ursprüngliche Form zurückzukehren.

Diese Maßnahme hat den Vorteil, daß die Einrichtung zum Greifen bzw. Durchtrennen besonders einfach hergestellt und betätigt werden kann, ohne daß Gelenke, Drehachsen oder ähnliches vorgesehen werden müssen. Die Biegung der zweiten, verschieblich gelagerten Backe kann oval oder abgeflacht sein oder auch kompliziertere Biegungen und Krümmungen aufweisen, je nach der gewünschten Anwendung.

In einer weiteren Ausgestaltung der Erfindung ist im Inneren der Hohlsonde ein Führungs- und Gleitelement für die zweite Backe vorgesehen.

Bei dieser Maßnahme ist vorteilhaft, daß das Verschieben der Backe und damit das Schließen bzw. Öffnen der Backe zum Greifen oder Loslassen reibungslos ausführbar ist. Das Führungs- und Gleitelement kann beispielsweise aus Teflon bestehen. Dies hat den weiteren Vorteil, daß ein unter Umständen auftretendes Verschweißen der zweiten Backe mit der Hohlsonde bei Ultraschallaktivierung, die mit Wärmeentwicklung einhergeht, sicher verhindert wird.

In einer weiteren vorteilhaften Ausgestaltung ist die zweite Backe der Einrichtung zum Greifen bzw. Durchtrennen durch Verschwenken von der ersten Backe abspreizbar.

Durch Verschwenken der Backe um eine Querachse wird die zweite Backe somit von der ersten Backe in eine offene Stellung der Einrichtung zum Greifen und/oder Durchtrennen weggeklappt bzw. in Richtung der ersten Backe wieder zugeklappt.

Somit ist es möglich, die zweite Backe vollständig von der distalen Öffnung der Hohlsonde wegzuklappen, so daß diese bei der Ausführung der Schneidefunktion mit der Schneideklinge bzw. beim Absaugen von Geweberesten nicht stört.

In einer weiteren vorteilhaften Ausgestaltung der Erfindung weist die Hohlsonde einen Außenschaft auf, an dem die zweite Backe verschwenkbar verankert ist.

Diese Maßnahme hat den Vorteil, daß der Außenschaft zur Befestigung bzw. Lagerung der zweiten Backe verwendet werden kann. Die Betätigung des Verschwenkens kann bspw. mit Stangen- bzw. Seilzug erfolgen, die auch innerhalb der Außenschaftwand vorgesehen werden können. Alternativ kann die Lagerung der zweiten Backe auch derart sein, daß ein Verschieben des Außenschafts das Verschwenken der zweiten Backe bewerkstelligt. Außerdem schützt der Außenschaft die Hohlsonde vorteilhafterweise vor mechanischen Beanspruchungen.

In dem Raum zwischen Außenschaft und Hohlsonde wird die Spülflüssigkeit zugeführt.

Durch das Vorsehen eines Außenschafts besteht außerdem die Möglichkeit, die Schneideklinge ganz in den Außenschaft einzuschieben und damit eine ungewollte Verletzung von Gewebe z.B. beim Einführen der Hohlsonde in den Körper zu vermeiden.

In einer weiteren Ausgestaltung der Erfindung besteht der Außenschaft aus einer oberen Hälfte und einer unteren Hälfte, die an einer Trennlinie entlang der Längsachse der Hohlsonde relativ zueinander verschiebbar sind, wobei ein Verschieben der Hälfte, an der die zweite Backe verankert ist, ein Verschwenken der zweiten Backe bewirkt.

Diese Maßnahme hat den Vorteil, daß das relative Verschieben der Außenschafthälften zueinander den notwendigen Zug auf die zweite Backe ausübt, um diese relativ zu der ersten Backe zu verschwenken.

In einer weiteren Ausgestaltung der Erfindung weist die Einrichtung zum Greifen und/oder Trennen ein Rohr mit einem Fortsatz auf, das im Innern der Hohlsonde aufgenommen ist, wobei Hohlsonde und Rohr relativ zueinander verdrehbar sind, und der Fortsatz arbeitet mit der Schneideklinge der Hohlsonde zusammen.

Diese Maßnahme hat den Vorteil, daß die Vorrichtung als Drehzange ausgebildet ist. Durch Drehen von Hohlsonde und Innenrohr relativ zueinander, dies kann dadurch erfolgen, daß entweder Innenrohr oder Außenrohr oder beide Rohre zueinander verdreht werden, können zwischen Fortsatz und Schneideklinge Gewebeteile gebracht werden und entweder ergriffen oder gar durchtrennt werden, so daß die Drehzange als Drehfaßzange oder als Drehschneidezange arbeiten kann.

Durch diese Geometrie wird eine besonders schlanke Bauweise gebildet, so daß keine über den äußeren Umfang des Außenschaftes seitlich vorspringenden Backen beim Öffnen vorhanden sind. Diese Ausgestaltung wird insbesonders dann eingesetzt werden, wenn relativ kleine Teile gefaßt bzw. aufgetrennt werden sollen.

In einer weiteren Ausgestaltung der Erfindung kommt der Fortsatz des inneren Rohres in einer Drehstellung eng anliegend mit der Schneideklinge zum Liegen, so daß beide Fortsätze als ein kompaktes Bauteil arbeiten.

Diese Maßnahme hat den Vorteil, daß in diesem Drehzustand mit der Hohlsonde so gearbeitet werden kann, als hätte sie keine Greifeinrichtung, da dann die beiden Rohre so zueinander verdreht sind, daß deren Fortsätze als ein Bauteil arbeiten. In dieser Drehstellung kann dann dieses kompakte Bauteil entweder als Schneideklinge und/oder auch als Spatel arbeiten.

In einer weiteren Ausgestaltung der Erfindung sind die Längskanten der Fortsätze stumpf.

Diese Maßnahme hat den Vorteil, daß dadurch die Zange gezielt als Faßzange arbeitet und kein Durchtrennen der gefaßten Teile erfolgt.

In einer weiteren Ausgestaltung der Erfindung sind die Längskanten der Fortsätze scharfkantig.

Diese Maßnahme hat den Vorteil, daß aufgrund dieser Ausgestaltung die Vorrichtung gezielt als Schneidezange arbeitet.

Es ist auch möglich diese beiden Ausgestaltungen mit einander zu koppeln, d.h. eine Kante scharf und eine Kante stumpf auszubilden, so daß in einer Drehrichtung lediglich eine Faßwirkung, in der anderen Drehrichtung eine Schneidewirkung erzielt wird.

In einer weiteren Ausgestaltung ist die Hohlsonde mit Hochfrequenzstrom (HF-Strom) beaufschlagbar.

Diese Maßnahme erlaubt der erfindungsgemäßen Vorrichtung die weitere Funktion, nämlich zur HF-Koagulation einsetzbar zu sein, also dazu, beispielsweise stark blutende Gefäße durch Anlegen von HF-Strömen schnell und effizient zu veröden.

Es versteht sich, daß hierzu ein Hochfrequenzgenerator notwendig ist, der den HF-Strom erzeugt. Der HF-Stom wird dann durch die Hohlsonde bis zu der proximalen Schneideklinge weitergeleitet und ruft in dem Gewebe, das mit der Schneideklinge in Verbindung steht, eine Denaturierung der Gewebebestandteile hervor.

Es versteht sich, daß die vorstehend genannten und die nachstehend noch zu erläuternden Merkmale nicht nur in den angegebenen Kombinationen, sondern auch in anderen Kombinationen oder in Alleinstellung verwendbar sind, ohne den Rahmen der vorliegenden Erfindung zu verlassen.

Bevorzugte Ausführungsbeispiele der erfindungsgemäßen Vorrichtung werden nachstehend unter Bezugnahme auf die Zeichnung erläutert. Es zeigen:
- Fig. 1: eine schematische Darstellung einer erfindungsgemäßen Vorrichtung zur Behandlung von Körpergewebe mittels Ultraschall;
- Fig. 2: eine stark vergrößerte Seitenansicht des distalen Endes eines ersten Ausführungsbeispiels einer Hohlsonde der erfindungsgemäßen Vorrichtung;
- Fig. 3: eine Draufsicht auf das distale Ende der Hohlsonde von Fig. 2;
- Fig. 4: einen Schnitt längs der Linie IV-IV in Fig. 2;
- Fig. 5: einen Längsschnitt durch das distale Ende eines weiteren Ausführungsbeispiels einer Hohlsonde einer erfindungsgemäßen Vorrichtung mit einer Einrichtung zum Greifen, und zwar in einer offenen Betriebsstellung der Greifeinrichtung;
- Fig. 6: einen Längsschnitt entsprechend Fig. 5, jedoch in einer geschlossenen Betriebsstellung der Greifeinrichtung;
- Fig. 7: eine Draufsicht auf das distale Ende eines weiteren Ausführungsbeispiels einer Hohlsonde einer erfindungsgemäßen Vorrichtung mit einer Greifeinrichtung in einer geschlossenen Betriebsstellung und mit einem Außenschaft;
- Fig. 8: eine Seitenansicht des distalen Endes entsprechend Fig. 7, und zwar in einer geschlossenen Betriebsstellung der Greifeinrichtung;
- Fig. 9: eine Seitenansicht entsprechend Fig. 8, jedoch in einer offenen Betriebsstellung der Greifeinrichtung;
- Fig. 10: einen Längsschnitt eines weiteren Ausführungsbeispiel eines distalen Endes einer Hohlsonde, die als Drehzange ausgebildet ist;
- Fig. 11: einen Schnitt längs der Linie XI-XI in Fig. 10, wobei die Fortsätze in diametral gegenüberliegender Position dargestellt sind;
- Fig. 12: einen der Fig. 11 vergleichbaren Schnitt, bei der der Fortsatz des inneren Rohres entgegen dem Uhrzeigersinn um ca. 45° verdreht ist;
- Fig. 13: eine den Fig. 11 und 12 vergleichbare Schnittdarstellung, bei der der Fortsatz des inneren Rohres um ca. 90° entgegen dem Uhrzeigersinn verdreht ist; und
- Fig. 14: eine der Darstellung der Fig. 11 bis 13 vergleichbare Darstellung, bei der der Fortsatz des inneren Rohres gegenüber der Darstellung von Fig. 11 um 180° gedreht ist und enganliegend auf der Schneideklinge der Hohlsonde zum Liegen kommt.

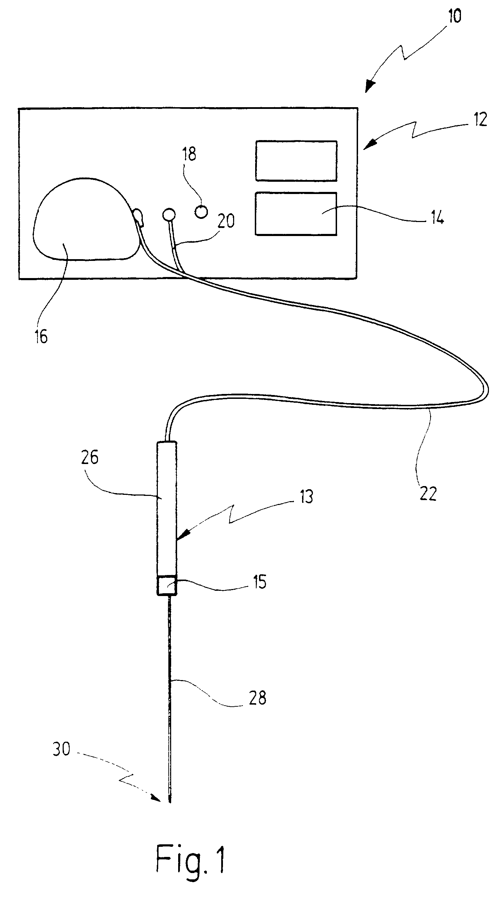

Fig. 1 zeigt eine Vorrichtung zur Behandlung von Körpergewebe mittels Ultraschall, die allgemein mit der Bezugsziffer 10 bezeichnet ist.

Die Vorrichtung 10 weist einen Generator 12 und eine Einrichtung 13 zum Übertragen des Ultraschalls auf Körpergewebe auf. Die Einrichtung 13 weist einen Ultraschallwandler 15 auf, der den Ultraschall erzeugt.

Der Generator 12 ist mit zwei Anzeigefeldern 14, einer peristaltischen Pumpe 16 und mit Buchsen 18 ausgestattet. Eine Versorgungsleitung 20 wird durch einen flexiblen Schlauch 22 geführt, der von der Pumpe 16 ausgeht. Der Schlauch 22 und die Versorgungsleitung 20 sind mit einem Handgriff 26 verbunden und enden in einer Hohlsonde 28.

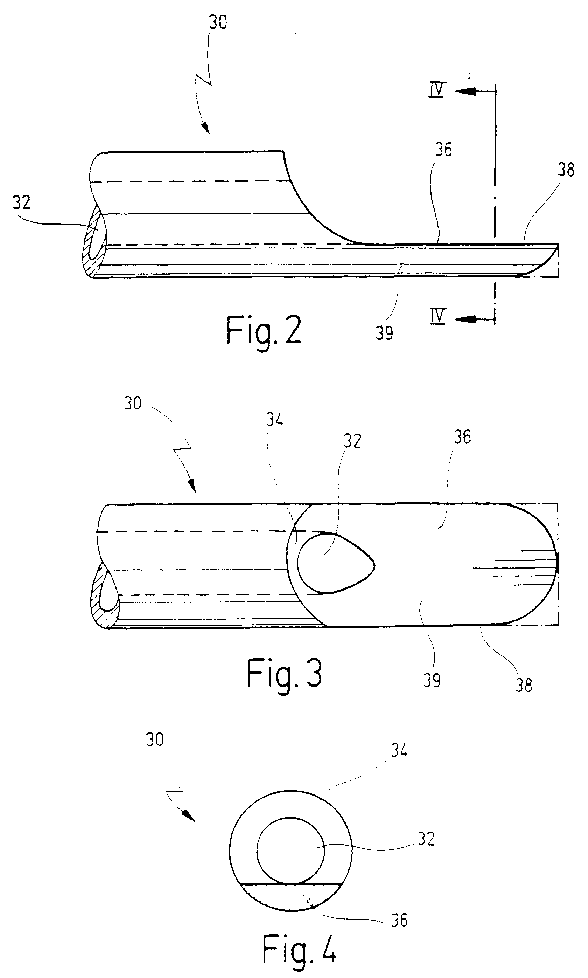

Die Hohlsonde 28 weist ein distales Ende 30 auf, dessen Ausführung in den Figuren 2 bis 4 besser ersichtlich ist.

Ein Spulmedium wird zwischen einem (hier nicht dargestellten) Außenschaft und der Hohlsonde 28 zum distalen Ende 30 geführt.

Die Hohlsonde 28 weist einen Kanal 32 zum Absaugen von Flüssigkeit und Gewebebestandteilen auf.

Die Hohlsondenwand 34 endet in einem ebenen flächigen Fortsatz 36, dessen Kanten 38 als Schneiden arbeiten, er stellt somit eine Schneideklinge 39 dar.

Beim Einsatz der erfindungsgemäßen Vorrichtung 10 wird der vom Ultraschallwandler 15 erzeugte Ultraschall, z.B. mit 24 KHz auf das distale Ende 30 der Hohlsonde 28 übertragen.

Durch den übertragenen Ultraschall wird insbesondere weiches, jedoch noch nicht festes Gewebe desintegriert und dabei verflüssigt, außerdem kommt es durch die ebenfalls durch den Ultraschall hervorgerufene Wärmeentwicklung zu einer Koagulation des Gewebes.

Gleichzeitig ruft der durch die Hohlsonde 28 geleitete Ultraschall eine Vibration des distalen Endes 30 der Hohlsonde 28 hervor. Durch diese Vibration wird die Schneideklinge 39 bewegt und zerschneidet damit effizient und schnell auch festes Gewebe.

Abschnittene und aufgelöste Gewebeteile werden direkt durch den Kanal 32 durch den mit der Pumpe 16 verbundenen Schlauch 22 abgesaugt.

In dem in den Figuren 2 und 3 gezeigten Ausführungsbeispiel weist die Schneideklinge 39 eine abgerundete scharfe Stirnkante auf. Diese kann, wie durch eine strichpunktierte Linie angedeutet auch eckig und stumpf sein.

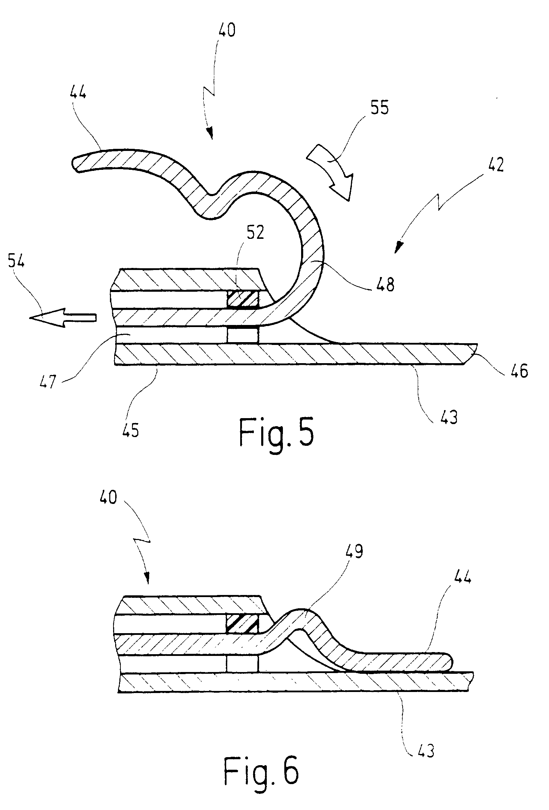

Die Figuren 5 und 6 beziehen sich auf eine weitere Ausführung einer Hohlsonde für die erfindungsgemäße Vorrichtung 10. Es ist nur das distale Ende der Hohlsonde gezeigt, das allgemein mit 40 bezeichnet ist.

Das distale Ende 40 weist eine Einrichtung zum Greifen 42 auf, die aus einer ersten Backe 43 und einer zweiten Backe 44 aufgebaut ist.

Die erste Backe 43 ist ein Fortsatz der Hohlsondenwand 45 und weist eine Schneideklinge 46 auf. Im Hohlsondenlumen 47 ist die aus einem superelastischen Material mit Formengedächtnis, einem sogenannten Memory-Metall, bestehende zweite Backe 44 verschieblich angeordnet. Das Memory-Metall ist bspw. eine Nickel-Titan-Legierung. Sie ragt aus dem Hohlsondenlumen heraus.

Die zweite Backe 44 wird im Hohlsondenlumen 47 durch ein aus Teflon bestehendes Gleit- und Führungselement 52 geführt.

In Fig. 5 ist eine geöffnete Betriebsstellung der Einrichtung zum Greifen 42 gezeigt. Die zweite Backe 44 ist weit aus dem Hohlsondenlumen 47 herausgeschoben und biegt sich aufgrund des Memory-Effekts unter Ausbildung einer Ausstülpung 48 nach oben und hinten um, wobei sie über das proximale Ende 40 herausragt.

Durch Verschieben der zweiten Backe 44 in das Hohlsondenlumen 47 hinein entlang der Richtung des Pfeils 54 wird die zweite Backe 44 verbogen und klappt dabei nach vorne in Richtung des Pfeils 55 um.

Durch weiteres Verschieben in Richtung des Pfeils 54 kommt die zweite Backe 44 auf der ersten Backe 43 zum Liegen. Diese Stellung ist in Fig. 6 gezeigt, es ist die geschlossene Betriebsstellung oder Greifstellung der Einrichtung 42 zum Greifen. In dieser Stellung können bspw. Gewebeschichten, Adern oder Nerven ergiffen und fest gehalten werden.

Ein Höcker 49 der zweiten Backe 44 bildet einen Anschlag, so daß ein völliges Einziehen der zweiten Backe 44 in das Hohlsondenlumen 47 hinein verhindert wird.

Trifft der Höcker 49 beim Einziehen auf die Hohlsondenwand wird er gestaucht und dadurch die Schließkraft verstärkt.

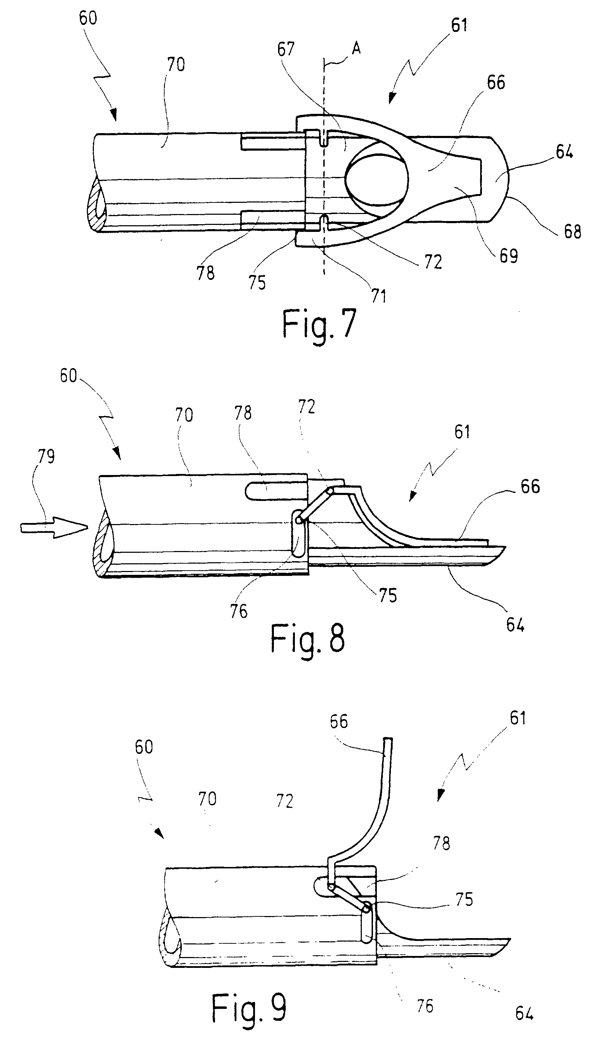

Ein weiteres Ausführungsbeispiel einer erfindungsgemäßen Hohlsonde mit Einrichtung zum Greifen ist in den Figuren 7 bis 9 gezeigt.

Ein distales Ende einer derartigen Hohlsonde ist allgemein mit 60 bezeichnet. An dem distalen Ende 60 ist eine Einrichtung 61 zum Greifen vorgesehen, die aus einer ersten Backe 64 und einer zweiten Backe 66 besteht. Die erste Backe 64 ist aus einem Fortsatz der Hohlsondenwand 67 gebildet und weist eine Schneideklinge 68 auf.

Die zweite Backe 66 besteht aus einer Zunge 69, von der proximal zwei Bügel 71 vorstehen, die die Hohlsondenwand 67 sowie einen Außenschaft 70, in dem das distale Ende 60 axial verschieblich gelagert ist, umgreifen.

Die Bügel 71 sind über Achsstifte 72 drehbar in entsprechenden Aufnahmen der Hohlsondenwand 67 befestigt. Außerdem weisen die Bügel 71 an ihren Enden radial nach innen stehende Zapfen 75 auf, die verschieblich in Kulissen 76 des Außenschafts 70 eingreifen.

Die Wand des Außenschafts 70 weist außerdem Aussparungen 78 auf, die sich im oberen Drittel des Außenschafts 70 entlang der Längsachse erstrecken.

In der in den Fig. 7 und 8 gezeigten geschlossenen Betriebsstellung der Einrichtung 61 zum Greifen liegt die Zunge 69 der zweiten Backe 66 flach auf der ersten Backe 64 auf.

Der Außenschaft 70 ist maximal nach proximal verschoben. Über den in der Kulisse 76 eingreifenden Zapfen 75 wird ein Anpressdruck auf die zweite Backe 66 ausübt, so daß ein Greifen und Festhalten von Gewebeteilen, Gefäßen o.ä. in dieser Stellung möglich ist.

Um die geschossene Betriebsstellung oder Greifstellung der Einrichtung 61 in eine um 90° geöffnete Betriebsstellung, die in Fig. 9 gezeigt ist, zu überführen, wird der Außenschaft 70 in Richtung des Pfeils 79 verschoben.

Der Zapfen 75 wandert in der Kulisse 76 und die zweite Backe 66 wird um ihre Drehachse A, die durch den Stift 72 definiert ist, nach oben verschwenkt. Der Stift 72 läuft dabei in die Aussparung 78 ein.

In der in Fig. 9 gezeigten Betriebsstellung ist die zweite Bakke 66 um 90° nach oben geklappt und gibt den Absaugkanal frei, so daß problemlos abgesaugt und gespült werden kann.

Durch entsprechende Ausgestaltung der Hebelgeometrie kann die zweite Backe 66 auch bis zu 180° verschwenkt werden, so daß diese längs der Außenseite des Außenschaftes angeschmiegt anliegt. In dieser Stellung kann die zweite Backe 66 verbleiben, falls zeitweilig keine Greiffunktion der Backen erwünscht oder notwendig ist.

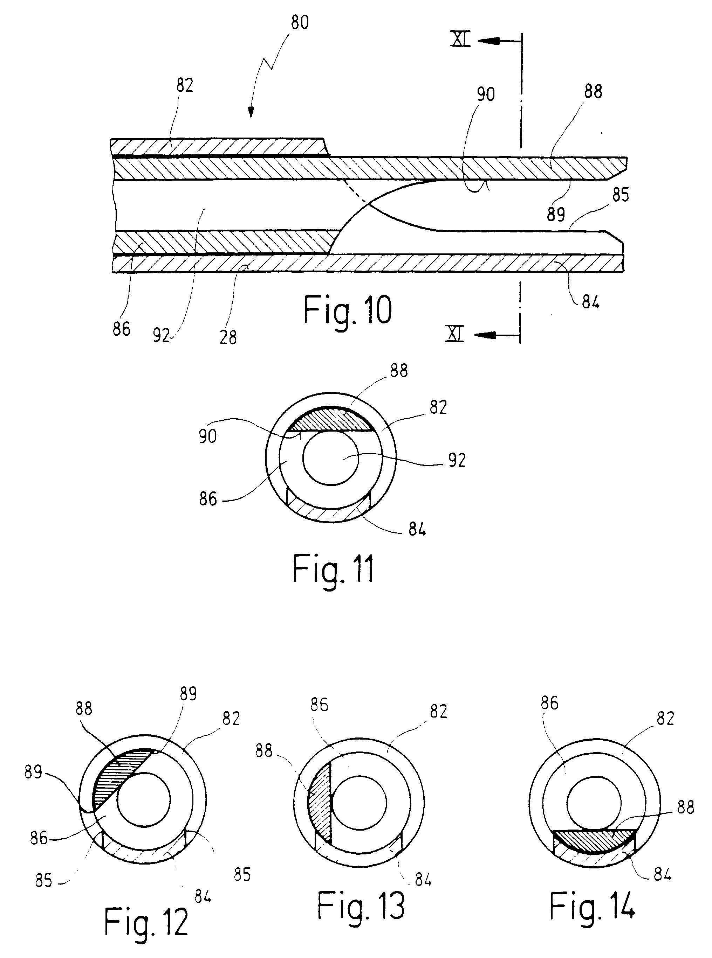

Bei dem in Fig. 10 bis 14 gezeigten weiteren Ausführungsbeispiel ist die Hohlsonde 28 am äußeren Ende mit einem Fortsatz 84 versehen. Der Fortsatz 84 ist dadurch entstanden, daß am distalen Ende 80 ein Teil der Rohrwand entfernt ist, so daß nur noch ein gewisser Umfangabschnitt der Rohrwand stehengeblieben ist, wie das insbesondere aus der Schnittdarstellung von Fig. 11 ersichtlich ist.

In die Hohlsonde 28 ist ein inneres Rohr 86 eingeschoben, dessen Außendurchmesser in etwa dem lichten Innendurchmesser der Hohlsonde 28 entspricht. Auch das innere Rohr weist am distalen Ende einen Fortsatz 88 auf, der dadurch entstanden ist, daß ein Teil der Rohrwand entfernt ist.

Im Gegensatz zu dem Fortsatz 84 ist der Schnitt jedoch so durchgeführt, daß dieser Fortsatz 88 eine ebene Innenseite 90 aufweist, wie das insbesondere aus der Schnittdarstellung von Fig. 11 ersichtlich ist. Der hohle Innenraum des inneren Rohrs 86 bildet einen Kanal 92 der zum Absaucen dient. In der Drehdarstellung von Fig. 10 und 11 steht der Fortsatz 84 diametral gegenüberliegend zum Fortsatz 88 des inneren Rohrs 86. In dieser Stellung kann die Hohlsonde 28 als Ultraschallaspiratorsonde betrieben werden.

Das innere Rohr 86 ist relativ zum Rohr 82 verdrehbar, wie das aus der Bildfolge von Fig. 11 zu Fig. 14 ersichtlich ist.

In dem in den Fig. 10 bis 14 dargestellten Ausführungsbeispiel weist der Fortsatz 84 scharfe Längskanten 85 auf, gleichermaßen weist der Fortsatz 88 scharfe Längskanten 89 auf.

Wird nun das innere Rohr 86 so verdreht wie es der Bildfolge von Fig. 11 zu Fig. 14 entspricht, wird ein zwischen den sich aufeinander zu bewegenden Kanten 89 und 85 gefaßtes Gewebestück aufgetrennt, somit arbeiten die beiden Fortsätze 84 und 88 als Schneiden.

Sind diese Kanten stumpf, können sie als Teile einer drehenden Faßzange arbeiten.

Aus Fig. 14 ist ersichtlich, daß in dieser Drehstellung die beiden Fortsätze 84 und 89 eng aneinander liegend sind und ein kompaktes Bauteil bilden, dessen Geometrie und Querschnitt in etwa dem in Fig. 4 dargestellten Fortsatz 36 entspricht, somit diese beiden Bauteile dann beispielsweise als Spatel arbeiten können.

Dies zeigt besonders deutlich die hohe Flexibilität und Multifunktionalität dieser Ausführung.

## Patentansprüche

1. Vorrichtung zur Behandlung von Körpergewebe mittels Ultraschall, mit einem Generator (12) und mit einer Einrichtung (13) zum Übertragen des Ultraschalls auf das Gewebe, die eine Hohlsonde (28) zum Absaugen des behandelten Gewebes aufweist, wobei im Bereich des distalen Endes (30; 40; 60; 80) der Hohlsonde (28) eine Schneideklinge (39) vorgesehen ist, **dadurch gekennzeichnet, daß** das distale Ende (30; 40; 60; 80) der Hohlsonde (28) eine Einrichtung (42; 61) zum Greifen und/oder Durchtrennen mit einer ersten und einer zweiten Backe (43, 44; 64, 66) aufweist.

2. Vorrichtung nach Anspruch 1, **dadurch gekennzeichnet, daß** die Schneideklinge (39) integraler Bestandteil der Hohlsondenwand (34; 45; 67) ist.

3. Vorrichtung nach Anspruch 2, **dadurch gekennzeichnet, daß** die Schneideklinge (39) als Fortsatz (36) der Hohlsondenwand (34; 45; 67) ausgebildet ist.

4. Vorrichtung nach Anspruch 1, **dadurch gekennzeichnet, daß** die erste Backe (43; 64) der Einrichtung (42; 61) zum Greifen und/oder Durchtrennen durch die Schneideklinge gebildet ist.

5. Vorrichtung nach Anspruch 4, **dadurch gekennzeichnet, daß** die zweite Backe (44) der Einrichtung (42) zum Greifen und/oder Durchtrennen entlang der Längsachse der Hohlsonde (28) verschiebar ist, aus einem superelastischen Material besteht und in einem aus der Hohlsonde (28) herausgeschobenen Zustand von ersten Backe (43) weggebogen ist und sich beim Hineinziehen in die Hohlsonde (28) zu der ersten Backe (43) hin bewegt.

6. Vorrichtung nach Anspruch 5, **dadurch gekennzeichnet, daß** an dem distalen Ende (40) im Inneren der Hohlsonde (28) ein Führungs- und Gleitelement (52) für die zweite Backe (44) vorgesehen ist.

7. Vorrichtung nach Anspruch 1 oder 4, **dadurch gekennzeichnet, daß** die zweite Backe (66) der Einrichtung (61) zum Greifen und/oder Durchtrennen durch Verschwenken von der ersten Backe (64) abspreizbar ist.

8. Vorrichtung nach Anspruch 7, **dadurch gekennzeichnet, daß** die Hohlsonde (28) einen Außenschaft (70) aufweist, an dem die zweite Backe (66) verschwenkbar verankert ist.

9. Vorrichtung nach Anspruch 8, **dadurch gekennzeichnet, daß** der Außenschaft (70) aus einer oberen Hälfte und einer unteren Hälfte besteht, die entlang der Längsachse der Hohlsonde (28) relativ zueinander verschiebbar sind, wobei ein Verschieben der Hälfte, an der die zweite Backe (44; 66) verankert ist, ein Verschwenken der zweiten Backe (44; 66) bewirkt.

10. Vorrichtung nach Anspruch 1, **dadurch gekennzeichnet, daß** die Einrichtung zum Greifen und/oder Trennen ein Rohr (86) mit einem Fortsatz (88) aufweist, das im Inneren der Hohlsonde (28) aufgenommen ist, wobei Hohlsonde (28) und Rohr relativ zueinander verdrehbar sind, und der Fortsatz (88) mit der Schneideklinge der Hohlsonde (28) im Sinne von zwei Backen zusammen arbeitet.

11. Vorrichtung nach Anspruch 10, **dadurch gekennzeichnet, daß** der Fortsatz (88) in einer Drehstellung auf der Schneideklinge der Hohlsonde (28) eng anliegend zum Liegen kommt (Fig. 14) und beide Fortsätze (84, 88) als ein kompaktes Bauteil arbeiten.

12. Vorrichtung nach Anspruch 10 oder 11, **dadurch gekennzeichnet, daß** die Längskanten (85, 89) der Fortsätze (84, 88) scharfkantig sind.

13. Vorrichtung nach Anspruch 10 oder 11, **dadurch gekennzeichnet, daß** die Längskanten der Fortsätze stumpf sind.

14. Vorrichtung nach einem der Ansprüche 1 bis 13, **dadurch gekennzeichnet, daß** die Hohlsonde (28) mit Hochfrequenz-Strom (HF-Strom) beaufschlagbar ist.

## Claims

1. Device for treating body tissue with ultrasound, comprising a generator (12) and means (13) for transmitting of the ultrasound to the tissue having a hollow probe (28) for suction of the treated tissue, a cutting blade (39) is provided in the region of the distal end (30; 40; 60; 80) of the hollow probe (28), **characterized in that** the distal end (30; 40; 60; 80) of the hollow probe is provided with means (42; 61) for grasping and/or cutting having a first and a second jaw (43, 44; 64, 66).

2. Device of claim 1, **characterized in that** the cutting blade (39) is an integral part of the wall (34; 45; 67) of the hollow probe.

3. Device of claim 2, **characterized in that** the cutting blade (39) is formed as a projection (36) of the wall (34; 45; 67) of the hollow probe.

4. Device of claim 1, **characterized in that** the first jaw (43; 64) of the means (42; 61) for grasping and/or cutting is formed by the cutting blade.

5. Device of claim 4, **characterized in that** the second jaw (44) of the means (42) for grasping and/or cutting is shiftable along the longitudinal axis of the hollow probe (28), and consists of a superelastic material and is bent away from the first jaw (43) in the state of being pushed out of the hollow probe (28), and moves toward the first jaw (43) when being drawn into the hollow probe (28).

6. Device of claim 5, **characterized in that** a guide and slide element (52) for the second jaw (44) is provided in the interior of the hollow probe (28) at its distal end (40).

7. Device of claim 1 or 4, **characterized in that** the second jaw (66) of the means (61) for cutting and/or grasping can be spread apart from the first jaw (64) by a swivelling movement.

8. Device of claim 7, **characterized in that** the hollow probe (28) comprises an outer shaft (70), at which the second jaw (66) is mounted swivelling.

9. Device of claim 8, **characterized in that** the outer shaft (70) consists of an upper half and a lower half, which are shiftable to one another along the longitudinal axis of the hollow probe (28), wherein a shifting of the half at which the second jaw (44; 66) is mounted causes a pivotable movement of the second jaw (44; 66).

10. Device of claim 1, **characterized in that** the means for cutting and/or grasping comprises a tube (86) having a projection (88), which is received in the interior of the hollow probe (28), whereby probe (28) and tube are rotatable relative to one another, and wherein the projection (88) interacts with the cutting blade of the hollow probe (28) in the sense of two jaws.

11. Device of claim 10, **characterized in that** the projection (88) comes tight in one angular position to the cutting blade of the probe (28) (Fig. 14) whereby the two projections (84, 88) act as a compact structure.

12. Device of claim 10 or 11, **characterized in that** the longitudinal edges (85, 89) of the projections (84, 88) are formed to be sharp.

13. Device of claims 10 or 11, **characterized in that** the longitudinal edges of the projections are to be blunt.

14. Device of anyone of claims 1 through 13, **characterized in that** the hollow probe (28) can be charged with high frequency current (HF-current).

## Revendications

1. Dispositif pour traiter des tissus corporels au moyen d'ultrasons, avec un générateur (12) et avec un équipement (13) pour transmettre l'ultrason sur le tissu, qui présente une sonde creuse (28) pour évacuer le tissu traité, sachant que dans la zone de l'extrémité distale (30 ; 40 ; 60 ; 80) de la sonde creuse (28) est prévue une lame de découpe (39), **caractérisé en ce que** l'extrémité distale (30 ; 40 ; 60 ; 80) de la sonde creuse (28) présente un équipement (42 ; 61) pour saisir et/ou séparer avec une première et une seconde mâchoire (43, 44 ; 64, 66).

2. Dispositif selon la revendication 1, **caractérisé en ce que** la lame de découpe (39) est un composant intégré de la paroi de sonde creuse (34 ; 45 ; 67).

3. Dispositif selon la revendication 2, **caractérisé en ce que** la lame de découpe (39) est formée comme appendice (36) de la paroi de sonde creuse (34 ; 45 ; 67).

4. Dispositif selon la revendication 1, **caractérisé en ce que** la première mâchoire (43 ; 64) de l'équipement (42 ; 61) pour saisir et/ou séparer est formée par la lame de découpe.

5. Dispositif selon la revendication 4, **caractérisé en ce que** la seconde mâchoire (44) de l'équipement (42) pour saisir et/ou séparer peut être déplacée le long de l'axe longitudinal de la sonde creuse (28), est fabriquée en matériau superélastique et est arquée en s'écartant de la première mâchoire (43) tout en sortant de la sonde creuse (28) et se déplace par traction dans la sonde creuse (28) jusqu'à la première mâchoire (43).

6. Dispositif selon la revendication 5, **caractérisé en ce qu'**à l'extrémité distale (40) à l'intérieur de la sonde creuse (28), un élément d'introduction et de glissement (52) est prévu pour la seconde mâchoire (44).

7. Dispositif selon la revendication 1 ou 4, **caractérisé en ce que** la seconde mâchoire (66) de l'équipement (61) pour saisir et/ou séparer peut être écartée par pivotement de la première mâchoire (64 ).

8. Dispositif selon la revendication 7, **caractérisé en ce que** la sonde creuse (28) présente une tige externe (70) à laquelle la seconde mâchoire (66) est ancrée de manière à pouvoir pivoter.

9. Dispositif selon la revendication 8, **caractérisé en ce que** la tige externe (70) est constituée d'une moitié supérieure et d'une moitié inférieure qui peuvent être déplacées l'une par rapport à l'autre le long de l'axe longitudinal de la sonde creuse (28), le déplacement de la moitié à laquelle la seconde mâchoire (44 ; 66) est ancrée entraînant le pivotement de la seconde mâchoire (44 ; 66).

10. Dispositif selon la revendication 1, **caractérisé en ce que** l'équipement pour saisir et/ou séparer présente un tube (86) avec un appendice (88), qui est placé à l'intérieur de la sonde creuse (28), la sonde creuse (28) et le tube pouvant tourner l'un par rapport à l'autre, et l'appendice (88) fonctionnant avec la lame de découpe de la sonde creuse (28) comme deux mâchoires.

11. Dispositif selon la revendication 10, **caractérisé en ce que** l'appendice (88), en une position rotative, vient reposer de manière collante sur la lame de découpe de la sonde creuse (28) (figure 14), les deux appendices (84, 88) fonctionnant comme un composant compact.

12. Dispositif selon la revendication 10 ou 11, **caractérisé en ce que** les bords longitudinaux (85, 89) des appendices (84, 88) sont tranchants.

13. Dispositif selon la revendication 10 ou 11, **caractérisé en ce que** les bords longitudinaux des appendices sont émoussés.

14. Dispositif selon l'une des revendications 1 à 13, **caractérisé en ce que** la sonde creuse (28) peut être alimentée par un courant à haute fréquence (courant HF).
